# EUROPEAN PATENT SPECIFICATION

(11) **EP 1 326 584 B1**
(45) Date of publication and mention of the grant of the patent: **02.09.2009**
(21) Application number: 01978708.4
(22) Date of filing: 18.10.2001
(51) Int. Cl.: A61K 9/00

(54) **DELIVERY SYSTEM AND METHOD OF TREATING OR PREVENTING OTITIS MEDIA**
ABGABESYSTEM UND VERFAHREN ZUR BEHANDLUNG ODER VORBEUGUNG VON MITTELOHRENENTZÜNDUNG
SYSTEME DISTRIBUTEUR ET METHODE DE TRAITEMENT OU DE PREVENTION DE L'OTITE MOYENNE

(30) Priority: 18.10.2000 DK 200001553
(43) Date of publication of application: 16.07.2003
(73) Proprietor: Auris Ehf., 220 Hafnarfirdi (IS)
(72) Inventor: SAEMUNDSDOTTIR, Gudrun, IS-220 Hafnarfirdi (IS)
(74) Representative: Arnason Faktor
(86) International application number: PCT/IB2001/001957
(87) International publication number: WO 2002/032394

(56) References cited:
- EP-A- 0 734 727
- WO-A-96/37210
- WO-A-97/01348
- US-A- 5 674 196
- EMILIO SANTOS, ET AL.: "Formulario Español de Farmacia Militar" , LABORATORIO Y PARQUE CENTRAL DE FARMACIA MILITAR , MADRID, ES XP002171650 page 30, last paragraph -page 31, paragraph 1

## Description

### TECHNICAL FIELD

The present invention relates to a delivery system and a method of treating or preventing otitis media.

### BACKGROUND

Otitis media is one of the most common childhood infections and a leading cause of pain and discomfort among children and distress with parents. The main pathogens are *Streptococcus* pneumonia and *Haemophilus influenzae.* The main antimicrobials used for treating otitis media are amoxycillin, amoxycillin with clavulanic acid and trimethoprim sulphamethoxazole. Otitis media is the single leading indication for antimicrobial prescriptions for children in the developed countries. During the last two decades, antimicrobial resistance among the most common bacteria causing otitis media has become widespread and highly prevalent in some areas. This has lead to problems in the medical treatment of the condition with failures becoming more frequent. Although it has been estimated that approximately three-quarter of otitis media cases resolve spontaneously, antimicrobial treatment is nevertheless necessary in its management, since it is often difficult or impossible to predict which children need antimicrobials. An alternative treatment not requiring the systematic administration of antimicrobials is therefore highly desirable.

Essential oils are commonly used in alternative medicine, and such treatment is then often referred to as aromatherapy. Common application means are skin oils and lotions (e.g. for use in massage), hot and cold compresses, hair care products (e.g. shampoos) and flower water, and common application methods are baths, vaporisation (e.g. in scent rooms and for inhalation), steam inhalation, douches, neat application and oral administration (internal use).

Essential oils are used in the alternative medicine for the treatment of various ailments. In the Encyclopedia of Essentail Oils (Julia Lawless, Element Books, Shaftesbury, 1992) it is stated that oil of Basil is suitable for the treatment of the following ailments: Insect bites, gout, muscular aches and pains, rheumatism, bronchitis, coughs, earache, sinusitis, dyspepsia, flatulence, nausea, cramps, scanty periods, colds, fever, flu, infectious diseases, anxiety, depression, fatigue, insomnia, migraine and nervous tension.

From handbooks on plant medicine it is known that Oil of Basil has anti-bacterial effect and that it may be used as ear drops in cases of ear infections.

From drug catalogues it is known as a general principle that local treatment of ear infections may be carried out by applying drops of a medicament or an ointment containing a medicament to a meche or a sponge.

However, the prior art methods of administering the drugs suffer from the drawback that the drug, e.g. essential oils, is in direct contact with the skin of the ear, which causes irritation and inflammation of the skin. Also, conventional medicines including prior art antimicrobials are not able to penetrate or diffuse through the intact tympanic membrane. Accordingly, physicians never prescribe external local treatment for otitis media.

EP-A2-734 727 discloses a pharmaceutical formulation for treating bacterial ear infections, the formulation containing about 85% by volume of Makadamia nut oil and 10 % by volume of tea tree oil, wherein 3 % by volume of Oil of Basil is added as an odorising agent.

WO 97/01348 discloses a pharmaceutical composition for treating e.g. aural infections comprising essential oils obtained by steam distillation of herbs from the genus origanum.

WO 96/37210 discloses a pharmaceutical composition containing etheric oils selected among a number of herbs. The composition is used as an anti-inflammatory agent for treatment of e.g. the ear.

Inouye et al. (27) (published after the priority date of the present invention) discloses the fact that the vapours of 14 essential oils have anti-bacterial activity.

### SUMMARY OF THE INVENTION

A first objective of the present invention is to provide an improved delivery system for administering a volatile substance to the ear.

This first objective is obtained by a delivery system adapted to be inserted into the external ear canal comprising a casing of a vapour impermeable material containing a volatile substance with therapeutic effect on otitis media, wherein the casing has a vapour-permeable opening.

The basis of the present invention is the finding that volatile compounds placed in the external ear canal will evaporate and can be brought to penetrate or diffuse through the tympanic membrane into the middle ear to combat the infection present there, and that it is possible to administer a therapeutic substance by this route in an amount sufficient to be treat otitis media effectively.

The invention is based on the first rationale that when using a vapour-impermeable casing for holding the drug, the direct contact between the drug and the skin of the ear may be avoided. Secondly, the invention is based on the rationale that when using a vapour-impermeable casing, which is closed at the exterior end and open at the interior end, it is possible to 1) direct the vapours in the direction of the tympanic membrane, 2) prevent evaporation of the vapours out of the ear and 3) increase the concentration of the vapours in the external ear canal to effect a strongly facilitated transport of vapours across the tympanic membrane into the middle ear.

Thus, the present invention has provided a possibility of treating otitis media locally in an effective manner.

A second objective of the present invention is to provide an improved method of treating or preventing otitis media.

This second objective is obtained with the method of the present invention comprising introducing into the external ear canal a volatile substance with a therapeutic effect on otitis media by means of the delivery system according to the invention.

The present invention further relates to the following:
- Oil of Basil for the treatment or prevention of otitis media.
- Use of Oil of Basil for the manufacture of a medicament for the treatment or prevention of otitis media.
- A pharmaceutical composition containing Oil of Basil as active ingredient.
- A pharmaceutical composition for treating or preventing otitis media containing Oil of Basil as active ingredient.
- Use of a pharmaceutical composition containing Oil of Basil as active ingredient for the manufacture of a medicament for treating or preventing otitis media.
- A pharmaceutical composition containing an effective dose of Oil of Basil as active ingredient.
- A pharmaceutical composition containing Oil of Basil as primary active ingredient.
- A pharmaceutical composition containing Oil of Basil as sole active ingredient.
- A pharmaceutical composition containing Oil of Basil as active ingredient excluding the composition disclosed in EP-A2-734 727: about 85 % by volume of Makadamia nut oil, 10 % by volume of tea tree oil, 3 % by volume of Oil of Basil and 2 % of an odorising agent.

It has surprisingly been discovered that oil of Basil has an antibacterial effect, and that oil of Basil is effective in treating otitis media when introduced into the external ear canal. It is believed that the mode of action of the oil of Basil is as follows: Volatile components of the oil of Basil evaporate from the surface onto which it is applied and penetrate through the tympanic membrane into the middle the ear to combat the bacteria present there.

### DETAILED DESCRIPTION OF THE INVENTION

### Delivery system

A preferred embodiment of the delivery system of the invention further comprises a carrier comprising the volatile substance.

Suitable as carrier in the delivery system of the present invention is any material or appliance, which can be introduced into the casing and be retained therein, and which can carry the volatile substance in such a way that the volatile substance is allowed to evaporate from the carrier.

Preferred carriers may e.g. be dressings, plasters, wads, pads, rubbers, sponges, strips. The carrier may be made of cotton etc.

In preparing the delivery system of the present invention, a suitable formulation of the volatile substance is introduced into the interior of the casing. As volatile substance formulation, any formulation mentioned below may be used.

When the formulation of volatile substance is solid or semi-solid, e.g. in the form of a paste or gel, it is preferably applied to the carrier or the wall of the casing in the form of a layer, from which evaporation of the volatile substance can take place.

When a liquid formulation of volatile substance is used, the formulation may be absorbed in a suitable carrier, e.g. a wad or a sponge. The wetted carrier is then placed in the interior of the casing. Alternatively, the liquid formulation is placed in the interior of the casing without having been absorbed on a carrier.

In order to avoid that the liquid formulation flows out from the casing, the vapour-permeable opening may be sealed off with a liquid-impermeable and vapour-permeable film.

The active components of the volatile substance formulation are preferably prevented from evaporating from the delivery system during storage by sealing off the vapour-permeable opening with a vapour-impermeable, removable film or lid. The said removable film may be made from any suitable vapour-proof material, such as a foil, e.g. an aluminium foil, and a plastic.

The delivery system of the invention is adapted to seal off the opening of the external ear canal to prevent or reduce the evaporation of the volatile substance from the external ear canal out into the surroundings, and hence to optimise the diffusion of the vapours of the volatile substance through the tympanic membrane into the middle ear. Such a seal may be in the form of the wall of the casing or it may have the form of a vapour-proof layer provided at the end of the casing to be placed at the opening of the external ear canal. The seal may be made from any vapour-proof material, such as a foil, e.g. an aluminium foil, and a plastic.

The casing is preferably made from rubber, plastic, silicone or related compounds.

In a preferred embodiment of the delivery system of the invention, the casing has one or more protrusions adapted to help keep the delivery system in place after insertion into the external ear canal. Preferably, the protrusion is an annular protrusion.

In another preferred embodiment of the delivery system of the invention, the casing has a tapered head at the end comprising the vapour-permeable opening. Such a tapered head both helps facilitate insertion of the delivery system into the ear and helps to keep it in place after insertion.

Preferably, the casing is oblong, in which case the vapour-permeable opening preferably is provided at one end of the casing. It is preferred that the casing has a circular cross-section.

### Method of treatment

The introduction of a therapeutic amount of volatile substance into the external ear canal is carried out using the delivery system of the invention, optionally comprising a carrier comprising the volatile substance, wherein the delivery system is placed in the said external ear canal.

The delivery system may be left in the external ear canal from 2 minutes to 24 hours, preferably from 5 minutes to 2 hours, more preferably from 10 minutes to 60 minutes, and most preferably from 20 to 40 minutes, e.g. 30 minutes.

When the duration of the treatment is between 20 and 40 minutes, the treatment is preferably repeated from every 4 to every 8 hours during the daytime, i.e. from 2-4 times a day. The treatment is continued until the earache has disappeared, usually 1-4 days. One last treatment is preferably given after the earache has disappeared.

### Volatile substance with therapeutic effect on otitis media

The volatile substance with therapeutic effect on otitis media may be any known antimicrobial substance, which is volatile. Such volatile antimicrobial substances include essential oils, alcohols, ketones, such as acetone, and ethers.

The volatile substance with therapeutic effect on otitis media may be any essential oil with antimicrobial properties. Essential oils from i.a. the following sources have been shown to have antimicrobial properties: Achillea fragrantissima (1), Alaska yellow cedar (13), Anise (7), Angelica (7,18), Basil (7,18), Bay (18), Bergamot (18), Cajeput (18), Calamintha nepeta (9), Camphor (24,18), Cardamon (2,7,18), Cassia (18), Celery (7,18), Chamomiles (18), Cinnamon (2,8,12,18), Cistus creticus (5), Clove (2,8,17,18), Coriander (7,17), Cumin (18), Dill (18), Dill weed (7), Fennel (7), Frankinsense (18), Eucalyptus (18), Geranium (15,16,17,18), Ho wood (18), Hoslundia opposita (10), Juniper heartwood (13), Ladano (6), Lavender (15,18), Lemon (16,18), Listerine (20,22), Litsea (18), Melissa (18), Marjoram (18), Myrrh (18), Myrtle (18), Nigella sativa (7), Neroli (18), Niaouli (18), Oregano (7), Orange (18,25), Palmorosa (18), Parsley (7), Patchouli (18), Peppermint (15,18,23), Petitgrain (18), Peumus boldus leaves (26), Pimento (18), Pineneedle (18), Piper angustifolium (24), Ravensera (18), Romarin oil (8), Rosemary (2,7,18), Rosewood (18), Sage (18,23), Schinus molle Linn (11), Senecio graveolens (Compositae) (21), Tanacetum parthenium (14), Tea tree oil (3,4,8,18,23), Thyme (17,18), Verbena (18), Western red cedar (13) etc.

Furthermore, the Encyclopedia of Essentail Oils (Julia Lawless, Element Books, Shaftesbury, 1992) mentions that essential oils from the following sources have antibacterial effect: Ajowan, Allspice, Amyris, Angelica, Anise star, Aniseed, Balm lemon, Balsam canadian, Balsam copaia, Balsam peru, Balsam tolu, Bay laurel Benzoin, Bergamot, Birch sweet, Birch white, Boldo leaf, Borneol, Bromm spanish, Buchu, Cabreuva, Cade, Cajeput, Calamintha, Calamus, Camphor, Cananga, Carrot seed, Cassia, Cassie, Cedarwood atlas, Cedarwood virginian, Celery seed, Chamomile german, Chamomile roman, Chervil, cinnamon, Citronella, Clove, Coriander, Costus, Cubebs, Cumin, Cypress, Deertongue, Dill, Elecampane, Elemi, Eucalyptus lemon-scented, Fennel, Fir needle silver, Frankincense, Galangal, Galbanum, Gardenia, Garlic, Geranium, Ginger, Grapefruit, Helichrysum, Horseradish, Hyacinth, Hyssop, Jaborandi, Jasmin, Juniper, Labdanum, Lavandin, Lavender spike, Lemongrass, Lime, Linaloe, Litsea cubeba, Lovage, Mandarin, Marigold, Marjoram sweet, Mastic, Mimosa, Mint, Mint peppermint, Mint spearmint, Mustard, Myrrh, Myrtle, Niaouli, Nutmeg, Oakmoss, Onion, Opopanax, Orange bitter, Orange blossom, Oregano common, Oregano Spanish, Palmarosa, Parsley, Patchouli, Rose, Rosemary, Rosewood, Rue, Sage clary, Sandalwood, Tea tree, Thyme common, Turpentine, Valerian, Verbena lemon, Vetiver, Violet, Yarrow, and Ylang ylang.

Preferred essential oils are oil obtained from Basil, Lavender, Chamomile, Tea tree and Sage, most preferable oil of Basil.

The essential oil suitable for use in the present invention may be prepared by extraction of plant material containing the essential oil. Any suitable extraction method may be used, including destillation, preferably steam destillation, expression, solvent extraction, enfleurage and maceration. Preferably, the extraction is carried out by steam distillation. Reference is made to the Encyclopedia of Essentail Oils (Julia Lawless, Element Books, Shaftesbury, 1992), page 35-37.

### Oil of Basil

The essential oil used as the volatile substance with therapeutic effect on otitis media is preferably oil of Basil (*Ocimum Basilicum*).

Any oil of Basil commercially available is suitable for use in the present invention.

The oil of Basil suitable for use in the present invention may be prepared by extraction of plant material containing oil of Basil. Preferably, the extraction is carried out by steam distillation.

The oil of Basil may be used in unmodified form, and it may be formulated as a pharmaceutical composition.

### Pharmaceutical composition

The pharmaceutical composition of the present invention may be formulated in any suitable formulation known in the prior art using any pharmaceutically acceptable excipients. The formulations include creams, ointments, lotions, liniments, gels, hydrogels, solutions, suspensions, sticks, sprays, pastes and plasters.

The pharmaceutically acceptable excipients may include emulsifying agents, antioxidants, buffering agents, preservatives, humectants, chelating agents, gel forming agents, ointment bases, perfumes and skin protective agents.

Examples of emulsifying agents are naturally occurring gums, e.g. gum acacia or gum tragacanth, naturally occurring phoshatides, e.g. soybean lecithin and sorbitan monooleate derivatives.

Examples of antioxidants are butylated hydroxy anisole (BHA), ascorbic acid and derivatives thereof, tocopherol and derivatives thereof, butylated hydroxy anisole and cysteine.

Examples of preservatives are parabens, such as methyl or propyl p-hydroxybenzoate and benzalkonium chloride.

Examples of humectants are glycerin, propylene glycol, sorbitol and urea.

Examples of chelating agents are sodium EDTA, citric acid and phosphoric acid.

Examples of gel forming agents are Carbopol, cellulose derivatives, bentonite, alginates, gelatin and polyvinyl pyrrolidone.

Examples of ointment bases are beeswax, paraffin, cetyl palmitate, vegetable oils, sorbitan esters of fatty acids (Span), polyethylene glycols, and condensation products between sorbitan esters of fatty acids and ethylene oxide, e.g. polyoxyethylene sorbitan monooleate (Tween).

Examples of skin protective agents include corticosteroids.

The formulation may include two or more volatile substances with therapeutic effect on otitis media, i.e. any mixture thereof.

### Definitions

In connection with the present invention, the term "Basil" means the herb "Ocimum Basilicum".

In connection with the present invention, the term "essential oil" means any aromatic product or extract obtained from natural sources, such as plants, including concretes, resinoids and absolutes, which contain a mixture of volatile and non-volatile components, such as a wax or resin.

In the following, the invention will be described in further detail with reference to the drawing.

### DRAWINGS

- Figure 1: shows one preferred embodiment of the delivery system according to the invention.
- Figure 2: shows another preferred embodiment of the delivery system according to the invention.
- Figure 3: shows the placement of a delivery system according to the invention in the external ear canal of a person.
- Figure 4: is a diagram showing the effect of treatment of otitis media caused by S. pneumoniae expressed as proportion improved vs. time after start of treatment.
- Figure 5: is a diagram showing the effect of treatment of otitis media caused by *H. influenzae* expressed as the proportion improved vs. time after start of treatment.

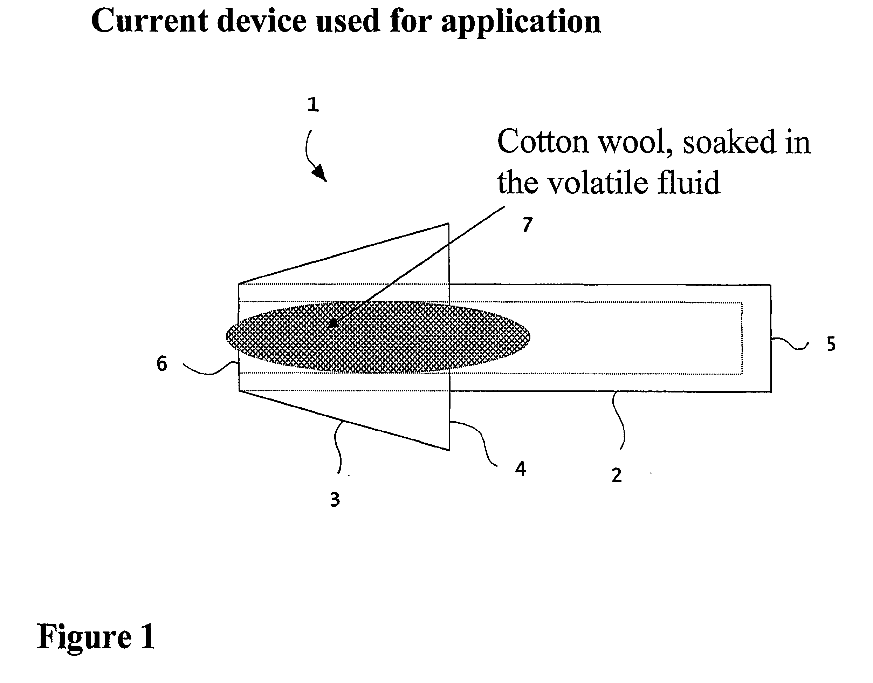

Figure 1 shows a delivery system 1 in the form of an ear plug comprising a casing 2 made from rubber or plastic with a tapered head 3 having an annular protrusion 4, which helps to keep the delivery system in place, once it has been inserted into external ear canal. The casing 2 is closed at its proximal end 5 and open at its distal end 6. The casing 2 contains a piece of cotton wool 7, which is soaked with e.g. 2-4 drops of volatile therapeutic liquid substance.

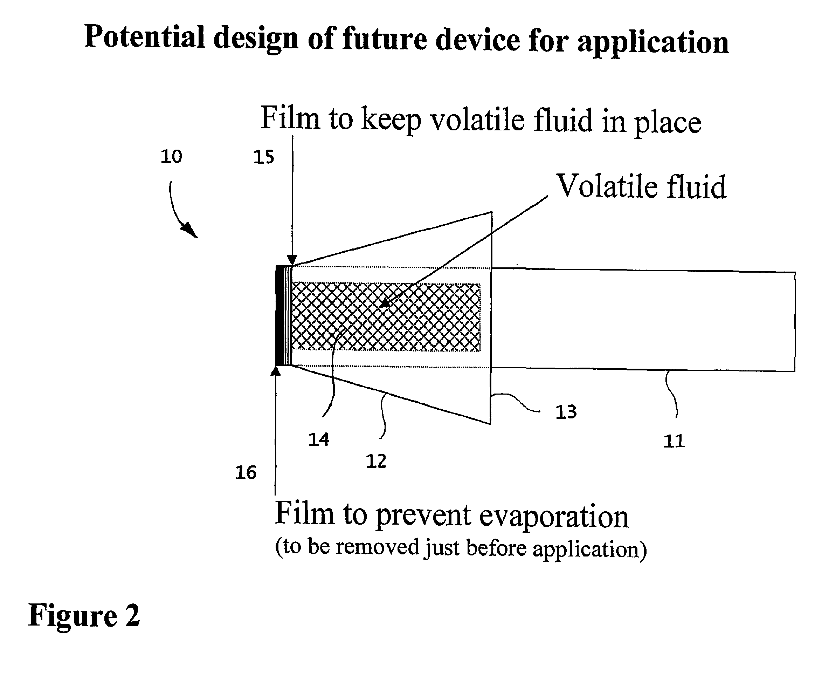

Figure 2 shows a delivery system 10 in the form of an ear plug comprising a casing 11 with a tapered head 12 having an annular protrusion 13, which helps to keep the delivery system in place, once it has been inserted into external ear canal. The casing 11 has a hollow space 14 in the distal part. The hollow space 14 contains a volatile therapeutic liquid substance. The hollow space 14 is sealed off at the distal end of the casing 11 with a sealing film 15, which is impermeable to liquid and open to penetration of vapours. On the external side of the sealing film 15, a removable vapour-proof covering layer 16 is placed to prevent evaporation of the therapeutic substance prior to use, wherein the covering layer 16 is to be removed immediately prior to use.

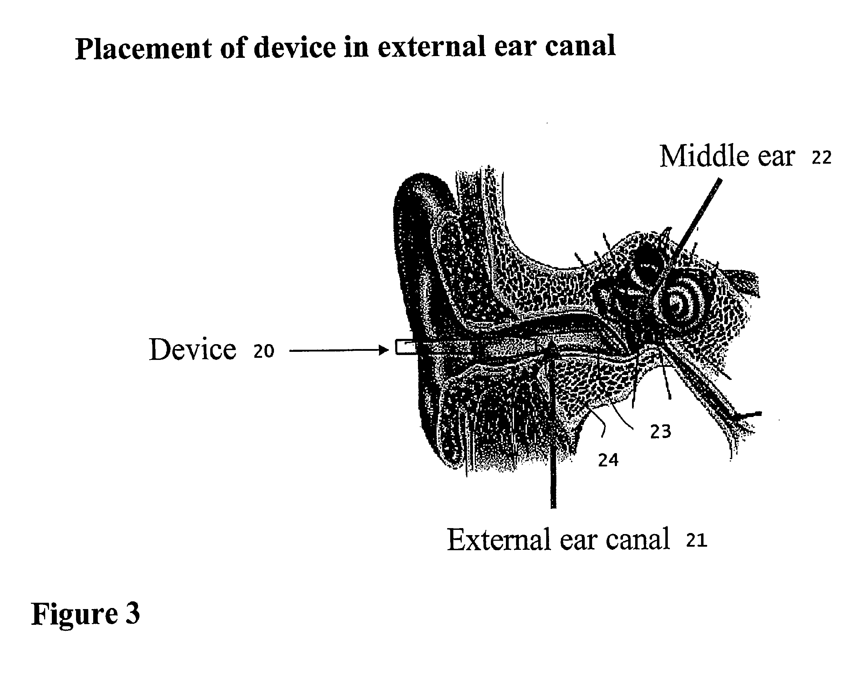

Figure 3 shows the placement of a delivery system 20 in the external ear canal 21 of a person. Figure 3 also shows the middle ear 22, where the bacterial infection to be treated is present, as well as the tympanic membrane 23 separating the external ear canal 21 and the middle ear 22. The volatile therapeutic liquid substance present in the delivery system 20 will evaporate from the distal end 24 of the delivery system 20 and penetrate the tympanic membrane 23 to reach the middle ear 22 and the bacteria present there, where it will serve its function as an antibacterial agent.

In the following, the invention will be described in further detail with reference to the examples.

### EXAMPLE

### Aim

To compare the efficacy of substance A and placebo, applied in the external ear canal, in the management of experimental otitis media in rats using experimental otitis media in rats induced by the leading pathogens, S. pneumoniae and *H. influenzae.* Substance A is basil oil with the trade name Aqua Oleum from Lower Wharf, Wallbridge, Stroud, Glos., GL5 3JA.

### Materials and methods

### Bacterial strains:

Three bacterial strains that had consistently caused otitis media in rats in previous experiments, were selected for the study. Two strains of S. *pneumoniae* (type 3, from Lund and type 6B (no. 9606+06001) from Iceland) and one of *H. influenzae* (non-capsulate, no. 3655 from Lund). The bacteria were stored at -70°C, subcultured from the storage medium (tryptic soy broth (Oxoid) with 10% glycerol) onto blood agar (S. *pneumoniae)* and chocolate blood agar (*H. influenzae*) plates. After 18 h incubation (with added CO₂) the S. *pneumoniae* strains were inoculated into Todd Hewitt broth (Oxoid), incubated for 6 h (until slightly turbid), harvested by centrifugation for 15 min at 3000 rpm and resuspended in fresh Todd Hewitt broth to an optical density corresponding to a concentration of 10⁶⁻⁷ cfu/ml. The H. influenzae strain was inoculated into Heart Infusion broth (Difco) with Fildes supplement (BBL), incubated for 3 hours (until slightly turbid), harvested by centrifugation for 15 min at 3000 rpm and resuspended in fresh Heart infusion broth (with Fildes supplement) to an optical density corresponding to a concentration of 10⁸ cfu/ml. Viable counts were performed to confirm the bacterial density and the suspensions were kept refrigerated (4°C) until used to inoculate the rats.

### Rat otitis media model

Healthy male Spraguea-Dawley rats, weighing 250-300 g were used. Before all operations, examination by an otomicroscope and insertion of substance A into the ear canals, the animals were anaesthetized with chloral hydrate administered intraperitoneally. Approximately 0.05 ml of the bacterial suspension was inoculated with a fine needle through the bony wall of the bulla directly into the middle ear cavity of the right ear as described earlier (Prellner et al., Microb Drug Resist 1999). Before inoculation the eardrums were inspected to confirm normal appearance.

The diagnosis of acute otitis media required direct visualization of opaque fluid behind the tympanic membrane and thickening of vessels. During inspections these symptoms were graded according to amount of fluid in the middle ear as F(+) to F+++ and according to the dilatation of vessels on the tympanic membrane as V(+) to V++, respectively (F=fluid, V=vessels). The fluid itself was characterized as either purulent, clear or mixed (both purulent and clear fluid visulized).

### Treatment and inspection schedule.

The experiments were performed on two separate occasions (part I and II). All rats were operated and inoculated with the appropriate bacterial suspension at day 0. They were inspected in the morning of day 2, and after inspection treatment was started with either substance A or placebo. Treatment was given again in the afternoon of day 2. Day 3 was just as day 2 with inspection and two treatments. The animals were all inspected again on day 4, and those that were not cultured by paracentesis were also inspected on day 5 and day 10. The treatment was given by soaking a small piece of cotton wool with either substance A or the placebo (olive oil). Rats treated with placebo are called controls. The cotton was placed in the outer ear canal of the right operated ear. The ear canal was subsequently sealed with a plug of plastic clay. Each treatment lasted until the rats had woken up well enough to remove the plug from the ear. This took from 1/2 to 1 hour. No rat had any cotton or clay left in the ear at the next inspection. After two days treatment (day 2 and 3), animals treated with substance A had all developed external otitis, and were not treated further.

Part I: A group of twenty five rats were inoculated with pneumococci (13 with type 6B and 12 with type 3) and another group of twenty five with the H. influenzae strain. In each group 17 animals received treatment with substance A and 8 placebo).

Part II: In order to exclude the possibility that otitis externa plays a role in the improvement of otitis media and to expand the pneumococcal experiments, fifty eight rats were inoculated with pneumococci (28 with type 6B and 30 with type 3). Sixteen rats in each group received treatment with substance A and 9 rats placebo(olive oil). External otitis was provoked by irritating the ear canal of 3 rats inoculated with type 6B and 5 with type 3.

### Culture of middle ear aspirates

Middle ear fluid fluid was sampled after needle paracentesis with a plastic inoculation loop and inoculated directly on blood agar with gentamicin (5 mg/l) (pneumococci) or chocolate blood agar (H. *influenzae*). These rats were sacrificed at the same time and were not available for further inspection.

Part I: At day 4, fluid from the right middle ear of 11 rats (3 controls and 8 treated) infected with pneumococci and 12 rats (4 controls and 8 treated) infected with H. influenzae, selected at random, was cultured. The remaining rats were sacrificed with their inner and middle ears intact for fixation in glutaraldehyde.

Part II: The middle ear fluid of all the animals was cultured at different times after the treatment was finished.

### Statistics

Healing rates were compared with the Fisher's exact test.

### Results

During part I, four rats (2 in the type 6B pneumococcal group and 2 in the H. influenzae group) developed cardiac arrest during the anaeshtesia and/or operation and died. The study groups in part I were therefore reduced to 23 rats.

### Pneumococcal infections

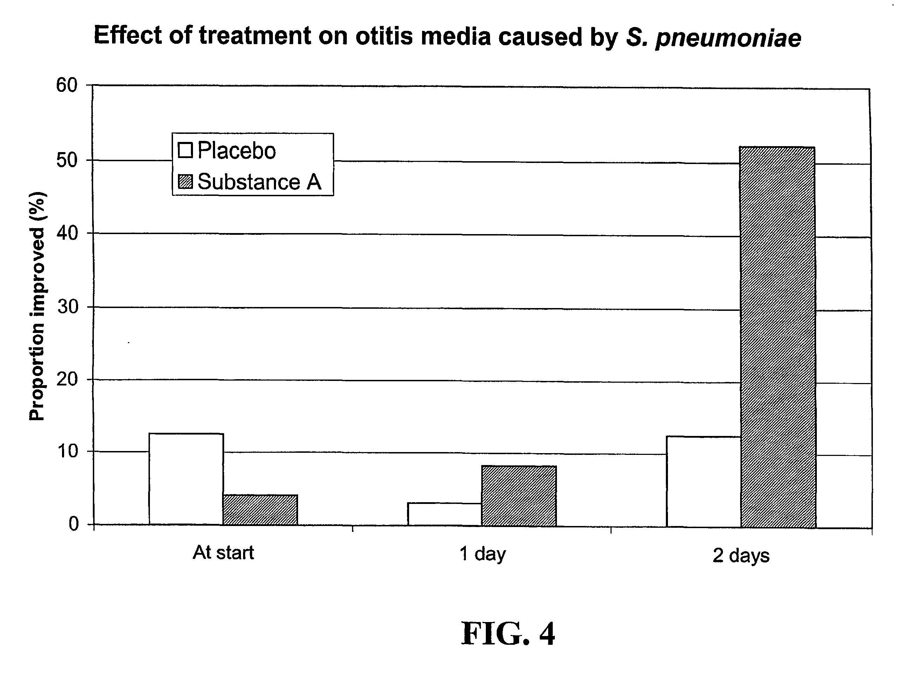

The results of the direct otomicroscopic inspections can be seen in table 1A. All animals had developed acute otitis media in the right ear on day 2, as judged by direct inspection. All animals in part I had purulent fluid and all but 6 in part II. These 6 rats had fluid that had started to become purulent but was still partly clear (mixed). On the third day, the otitis was still purulent in all rats in part I, and all but one of the animals that had mixed appearance on day 2 had now developed purulent fluid. After treatment, on the fourth day, 25 rats in the treatment group had improved, i.e. the fluid had become completely clear or had started to become clear but was still partly purulent (mixed). Only 3 of the rats in the control group showed signs of such improvement on the fourth day after inoculation, i.e. day 2 after start of treatment (p=0.0017) (Figure 4). After the third treatment with substance A, all rats developed otitis externa. The inflammation and exudation prevented adequate examination of the eardrum in some of the other animals on day 5. The results of the middle ear cultures can be seen in table 1A. The difference in culture rates between the treatment and control groups is not significant (p=1).

**Table 1A Streptococcus pneumoniae**

| | | | | | | | | |
|---|---|---|---|---|---|---|---|---|
| Results of otomicroscopic examination of the infected right ear on days 2, 3 and 4. The amount of fluid is graded as -/(+)/+/++/+++, the expansion of vessels as -/(+)/+/++ (V=vessels) and the results of culture as NG /(+)/+/++/+++ (NG=no growth of pneumococci; - =not done). | | | | | | | | |

| **Group** | | **Day 2** | | **Day 3** | | **Day 4** | | |
|---|---|---|---|---|---|---|---|---|
| **Control** | **Part** | **Fluid** | **V** | **Fluid** | **V** | **Fluid** | **V** | **Culture** |
| type 3 | I | + pus | + | + pus | + | ++ pus | + | - |
| type 3 | I | + pus | + | +++pus | + | +++ pus | (+) | NG |
| type 3 | I | (+) pus | (+) | ++ pus | + | +++ pus | (+) | + I |
| type 3 | II | ++mixed | + | ++ pus | ++ | + pus | ++ | (+) |
| type 3 | II | +++ pus | + | +++pus | ++ | +++ pus | ++ | + |
| type 3 | II | + mixed | + | + pus | + | + pus | + | NG |
| type 3 | II | +++ pus | + | +++pus | + | ++ pus | + | + |
| type 3 | II | +++ pus | + | +++pus | + | +++mixed | + | (+) |
| type 3 | II | +++ pus | + | +++pus | + | +++ pus | + | ++ |
| type 3 | II | ++ pus | + | ++ pus | + | ++ pus | + | NG |
| type 3 | II | +++ pus | + | +++pus | + | ++ pus | ++ | NG |
| type 3 | II | ++ pus | + | ++ pus | + | ++ pus | + | + |
| Type6B | I | + pus | + | + pus | + | + pus | + | - |
| Type6B | I | + pus | + | + pus | + | + pus | + | NG |
| Type6B | I | ++ pus* | + | ++pus* | + | + pus* | + | - |
| Type6B | II | ++mixed | + | ++ pus | + | + pus | + | NG |
| Type6B | II | ++ pus | ++ | ++ pus | + | ++ pus | + | NG |
| Type6B | II | +++ pus | + | +++pus | + | ++ pus | ++ | NG |
| Type6B | II | ++ pus | ++ | ++ pus | + | + clear | + | NG |
| Type6B | II | ++mixed | + | +++mixed | + | + pus | + | NG |
| Type6B | II | + pus | ++ | ++ pus | ++ | + clear | + | NG |
| Type6B | II | ++ pus | ++ | +++pus* | + | +++ pus* | + | + |
| Type6B | II | ++ pus | + | +++ pus | + | +++ pus | ++ | NG |
| Type6B | II | ++ pus | + | ++ pus | + | ++ pus | + | NG |

| **Ext. otitis** | | | | | | | | |
|---|---|---|---|---|---|---|---|---|
| type 3 | II | ++ pus | + | ++ pus | + | ++ mixed | + | NG |
| type 3 | II | + pus | + | ++ pus | + | ++ pus | ++ | + |
| type 3 | II | ++ pus | + | ++ pus | + | ++ pus* | ? | NG |
| type 3 | II | ++ pus* | + | ++pus * | + | ++ pus* | ++ | ++ |
| type 3 | II | (+) pus | + | (+) pus | + | + pus | + | NG |
| type6B | II | +++ pus | ++ | ++ pus | + | + pus | + | NG |
| type6B | II | + pus | ++ | ++ pus | + | + pus* | + | NG |
| type6B | II | +++ pus | + | +++ pus | + | ++ pus | + | NG |

| **Treatment** | | | | | | | | |
|---|---|---|---|---|---|---|---|---|
| type 3 | I | ++ pus | + | ++ pus | + | + pus | ++ | NG |
| type 3 | I | ++mixed | + | + pus | + | + mixed | ++ | - |
| type 3 | I | ++ pus | ++ | ++ pus | + | ++ pus | + | NG |
| type 3 | I | ++ pus | + | + pus | + | + mixed | ++ | (+) |
| type 3 | I | +++ pus | + | ++ pus | + | ++ mixed | ++ | - |
| type 3 | I | ++ pus | + | ++ pus | + | ++ mixed | + | + |
| type 3 | I | + pus | + | + pus | + | ++ mixed | ++ | - |
| type 3 | I | + pus | + | + pus | + | + mixed | ++ | - |
| type 3 | I | ++ pus | + | + pus | + | + mixed | ++ | - |
| type 3 | II | ++ | ++ | ++ pus | + | +++ pus* | + | NG |
| type 3 | II | +++pus* | + | ++ pus* | + | ++ pus* | ? | +++ |
| type 3 | II | ++ pus | + | ++mixed | + | +++ pus | ++ | NG |
| type 3 | II | ++ pus | + | ++ pus | + | ++ pus* | ++ | NG |
| type 3 | II | ++ pus | + | ++ pus | + | ++ pus | ++ | (+) |
| type 3 | II | +++ pus | + | +++ pus | + | +++ pus | + | ++ |
| type 3 | II | ++ pus | + | ++mixed | + | + mixed | + | NG |
| type 3 | II | ++ pus | + | +++ pus | + | +++ pus | + | (+) |
| type 3 | II | +++ pus | + | ++mixed | + | +++ pus* | + | ++ |
| type 3 | II | +++ pus | + | +++pus | + | +++pus* | + | +++ |
| type 3 | II | ++ pus | ++ | ++ pus | + | ++ pus | + | (+) |
| type 3 | II | +++ pus | ++ | ++mixed | + | +++mixed | ++ | (+) |
| type 3 | II | ++ pus* | + | ++pus* | + | | | + |
| type 3 | II | ++ pus* | + | ++pus* | + | +++pus* | + | NG |
| type 3 | II | ++ pus* | + | ++pus* | + | ++ pus* | ? | NG |
| type 3 | II | ++mixed | + | + pus | + | ++ pus* | ? | NG |
| Type6B | I | ++ pus | + | ++ pus | + | ++ pus | ++ | NG |
| Type6B | I | + pus | + | + pus* | + | + pus* | + | - |
| Type6B | I | + pus | + | ++ pus | + | ++mixed | ++ | NG |
| Type6B | I | ++ pus | + | ++ pus | + | ++mixed | ++ | (+) |
| Type6B | I | ++ pus | + | + pus | + | ++ pus | ++ | + |
| Type6B | I | + pus | (+) | + pus | + | ++mixed | ++ | - |
| Type6B | I | ++ pus | + | ++ pus | + | ++mixed | ++ | - |
| Type6B | I | + pus | + | + pus | + | + mixed | + | - |
| Type6B | II | +++ pus | + | ++ pus | ++ | + mixed | + | NG |
| Type6B | II | +++ pus | ++ | +++pus | + | + pus | ++ | (+) |
| Type6B | II | +++ pus | ++ | +++pus | + | + mixed | + | NG |
| Type6B | II | ++ pus | + | ++ pus | + | + pus | + | NG |
| Type6B | II | ++ pus | + | ++ pus | + | + clear | ++ | NG |
| Type6B | II | ++ pus | + | +++pus | + | ++mixed | + | NG |
| Type6B | II | ++ pus | ++ | +++pus | + | ++ pus | + | +++ |
| Type6B | II | +++ pus | + | +++pus* | ++* | ++ pus* | ? | NG |
| Type6B | II | ++ pus | + | + pus | + | + mixed | + | NG |
| Type6B | II | ++ pus | ++ | + pus | + | + clear | + | NG |
| Type6B | II | ++ pus | + | ++ pus | + | ++mixed | + | NG |
| type6B | II | ++ pus | ++ | ++ pus | + | ++clear | + | NG |
| Type6B | II | ++ pus | + | + pus | + | + mixed | + | NG |
| Type6B | II | +++ pus | ++ | + pus | + | + mixed | + | NG |
| Type6B | II | +++ pus | + | ++ pus | + | + mixed | + | NG |
| type 6B | II | ++ pus | ++ | ++ pus | + | ++ pus | + | NG |

| | | | | | | | | |
|---|---|---|---|---|---|---|---|---|
| *The eardrum was perforated. | | | | | | | | |

### Haemophilus influenzae infections.

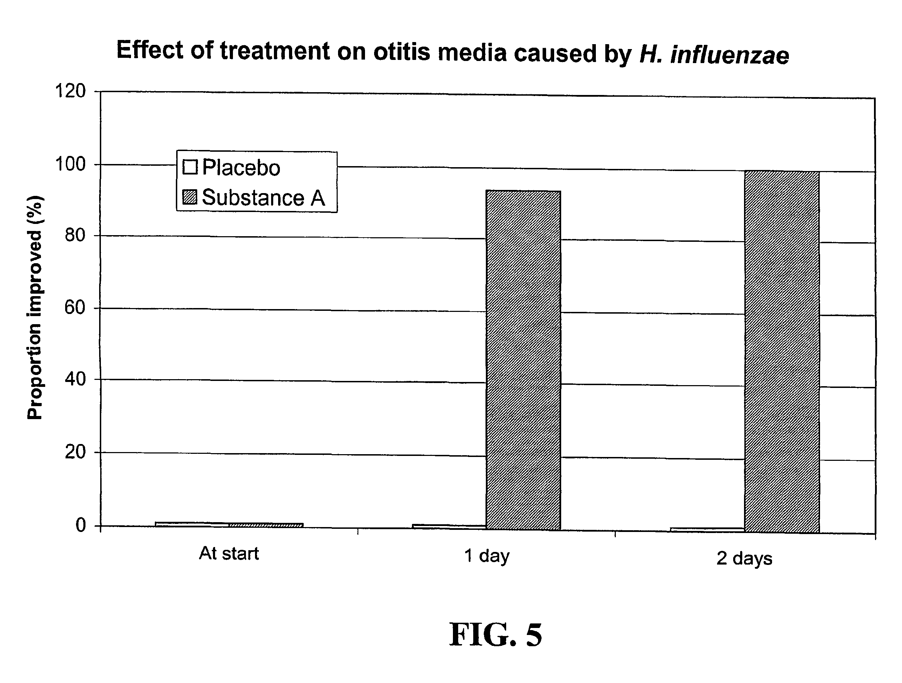

The results of the direct otomicroscopic inspection can be seen in table 1B. All animals had developed acute purulent otitis media in the right ear on day 2, as judged by direct inspection. The otitis had already started clearing in the treated group on day 3, but there was no improvement in the control group until day 5. On day 3, 4 of the rats treated with substance A had cleared the fluid of purulence in the middle ear, but none of the control animals (p= 0,27). This difference is not statistically significant. By looking at improvement (i.e. purulence cleared or started to clear), 15 treated rats had improved but none of the control rats (p<0.0001). On day 4 after inoculation (day 2 after start of treatment), all but 3 treated rats no longer had purulent otitis, but all the control rats (p=0.0005) (Figure 5). After day 4, further evaluation was not useful, since paracentesis was performed on 11 of the rats on day 4. Just like for the treatment of the pneumococcal infections, all rats developed otitis externa after the third treatment with substance A. However, the inflammation and exudation did not prevent adequate examination of the eardrum in these animals. Culture from the middle ear fluid of 11 rats on day 4, yielded growth of H. influenzae from 3 animals, all in the control group but none from the treatment group (see table 1B) (p=0.02).

**Table 1B Haemophilus influenzae**

| | | | | | | | |
|---|---|---|---|---|---|---|---|
| Results of otomicroscopic examination of the infected right ear on days 2, 3 and 4. The amount of fluid is graded as -/(+)/+/++/+++, the expansion of vessels as -/(+) /+/++ (V=vessels) and the results of culture as NG/(+)/+ /++/+++ (NG=no growth of H. influenzae; - =not done). | | | | | | | |

| **Group** | **Day 2** | | **Day 3** | | **Day 4** | | |
|---|---|---|---|---|---|---|---|
| **Control** | **Fluid** | **V** | **Fluid** | **V** | **Fluid** | **V** | **Culture** |
| | + pus | + | ++ pus | ++ | ++ pus | + | (+) |
| | (+)pus | (+) | + pus | + | + pus | + | (+) |
| | ++ pus | + | ++ pus | + | ++ pus | + | - |
| | ++ pus | + | ++ pus | + | ++ pus | + | NG |
| | ++ pus | + | ++ pus | + | ++ pus | + | (+) |
| | ++ pus | + | ++ pus | + | ++ pus | + | - |
| | ++ pus | + | ++ pus | + | ++ pus | + | - |

| **Treatment** | | | | | | | |
|---|---|---|---|---|---|---|---|
| | ++ pus | ++ | ++mixed | ++ | ++mixed | + | - |
| | ++ pus | ++ | ++mixed | + | + clear | + | - |
| | + pus | + | ++mixed | ++ | + mixed | + | - |
| | +++pus | + | ++mixed | ++ | ++mixed | ++ | - |
| | ++ pus | + | + pus | + | + clear | + | - |
| | ++ pus | + | + mixed | ++ | + clear | + | NG |
| | ++ pus | + | + clear | + | + clear | + | - |
| | ++ pus | + | ++mixed | ++ | + clear | + | NG |
| | + pus | + | + mixed | ++ | + clear | + | NG |
| | ++ pus | + | ++mixed | ++ | + clear | + | NG |
| | + pus | + | + clear | ++ | + clear | + | - |
| | ++ pus | + | + mixed | + | + clear | + | NG |
| | ++ pus | + | ++mixed | + | + clear | + | - |
| | ++ pus | + | + clear | ++ | + clear | + | NG |
| | ++ pus | + | ++clear | + | + clear | + | - |
| | ++ pus | + | ++mixed | + | + clear | + | NG |

### Discussion

Treatment with substance A, given externally in the ear canal, leads to significantly better healing and improvement of *H. influenzae* experimental otitis media than placebo. In addition, it leads to an improvement in significantly more otitis media cases infected with pneumococci, and showed a clear tendency for greater healing, than placebo. Bacteriological cure rates did not differ between the treated and control groups infected with pneumococci, but treatment with substance A resulted in significantly greater bacteriological cure rates than placebo in H. influenzae infected rats. This is the first time that application of local external treatment for otitis media leads to significantly better results than placebo.

Unfortunately, the treatment with substance A was associated with the development of external otitis, after the third treatment. The treatment was therefore not continued beyond the second treatment day. The treatment with substance A could possibly have resulted in further healing if it had been continued. The possibility of a healing effect of external otitis media could not be excluded. Although, this was considered an unlikely possibility, external otitis was induced in 8 rats infected with pneumococci. The external otitis did not show any effect on the middle ear infection.

The results of this study show that external treatment with substance A is significantly more effective in improving and healing otitis media caused by H. influenzae, and significantly more effective in improving otitis media caused by pneumococci, than placebo. This treatment approach may become an important alternative treatment for acute otitis media, since it does not require systemic use of antimicrobials.

### LIST OF REFERENCES

(1) Barel S, Segal R, Yashphe J. The antimicrobial activity of te essential oil from Achillea fragrantissima. J Ethnopharmacol 1991;33:187-91.
(2) Calcuttawalla A, Bhatt RM, Pandita N, Vaidya ADB. Potent antimicrobial activity of volatile oils from some spices. Abstract, 99th Annual Meeting, American Society for Microbiology, Chicago, May 1999. ASM, Washington, 1999.
(3) Carson CF, Riley TV. Antimicrobial activity of the major components of the essential oil of Melaleuca alternifolia. J Appl Bacteriol 1995;78:264-9.
(4) Cox SD, Mann CM, Markham JL, Bell HC, Gustafson JE, Warmington JR, Wyllie SG. The mode of antimicrobial action of the essential oil of Melaleuca alternifolia (tea tree oil). J Appl Microbiol 2000;88:170-5.
(5) Demetzos C, Katerinopoulos H, Kouvarakis A, Stratigakis N, Loukis A, Ekonomakis C, Spiliotis V, Tsaknis J. Composition ant antimicrobial activity of the essential oil of Cistus creticus subsp. eriocephalus [letter]. Planta Med 1997;63:477-9.
(6) Demetzos C, Stahl B, Anastassaki T, Gazouli M, Tzouvelekis LS, Rallis M. Chemical analysis and antimicrobial activity of the resin Ladano, of its essential oil and of the isolated compounds [letter]. Planta Med 1999;65:76-8.
(7) Elgayyar M, Draughon FA, Golden DA, Mount JR. Antimicrobial activity of essential oils from plantsw to selected pathogenic and saprophytic microorganisms. Abstract, 99th Annual Meeting, American Society for Microbiology, Chicago, May 1999. ASM, Washington, 1999.
(8) Favre-Bonte S, Kamel C, Joly B, Forestier C. Antimicrobial properties of essential oils and spices extracts ageinst food pathogens. Abstract, 99th Annual Meeting, American Society for Microbiology, Chicago, May 1999. ASM, Washington, 1999.
(9) Flamini G, Cioni PL, Puleio R, Morelli I, Panizzi L. Antimicrobial activity of the essential oil of Calamintha nepeta and its constituent pulegone against bacteria and fungi. Phytother Res 1999;13:349-51.
(10) Gundidza GM, Deans SG, Svoboda KP, Mavi S. Antimicrobial activity of essential oil from Hoslundia opposita. Centr Afr J Med 1992;38:290-3.
(11) Gunidza M. Antimicrobial activity of essential oil from Schinus molle Linn. Centr Afr J Med 1993,39:231-4.
(12) Hili P, Evans CS, Veness RG. Antimicrobial action of essential oils: the effect of dimethylsulphoxide on the activity of cinnamon oil. Lett Appl Microbiol 1997;24:269-75.
(13) Johnston WH, Karchesy J, Craig AM. Antimicrobial activity of heartwood extracts and essential oils derived from several tree species against anaerobic bacteria and yeast. Abstract, 99th Annual Meeting, American Society for Microbiology, Chicago, May 1999. ASM, Washington, 1999.
(14) Kalodera Z, Pepeljnjak S, Blazevic N, Petrak T. Chemical composition and antimicrobial activity of Tanacetum parthenium essential oil. Pharmazie 1997;52:885-6.
(15) Lis-Balchin M, Hart S. Studies on the mode of action of the essential oil of lavender (Lavundula angustifolia, P. Miller). Phytother Res 1999;13:540-2.
(16) Lis-Balchin M; Buchbauer G; Ribisch K; Wenger MT. Comparative antibacterial effects of novel Pelargonium essential oils and solvent extracts. Lett Appl Microbiol 1998;27(3):135-41.
(17) Lis-Balchin M, Buchbauer G, Hirtenlehner T, Resch M. Antimicrobial activity of Pelargoinium essential oils added to a quiche filling as a model food system. Lett Appl Microbiol 1998;27:207-10.
(18) Lis-Balchin M, Deans SG. Bioactivity of selected plant essential oils against Listeria monocytogenes. J Appl Microbiol 1997;82(6):759-62.
(19) Lis-Balchin M, Deans SG. Antimicrobial effects of hydrophilic extracts of Pelargonium species (Geraniaceae). Lett Appl Microbiol 1996;23(4):205-7
(20) Pan PH, Finnegan MB, Sturdivant L, Barnett ML. Comparative antimicrobial activity of an essential oil and an amine fluoride/stannous fluoride mouthrinse in vitro. J Clin Periodontol 1999;26:474-6.
(21) Perez C, Agnese AM, Cabrera JL. The essential oil of Senecio graveolens (Compositae): chemical composition and antimicrobial activity tests. J Ethnopharmacol 1999;66:91-6.
(22) Riep BG, Bernimoulin JP, Barnett ML. Comparative antiplaque effectiveness of an essential oil and an amine fluoride/stannous fluoride mouthrinse. J Clin Periodontol 1999;26:164-8.
(23) Shapiro S, Meier A, Guggenheim B. The antimicrobial activity of essentaila oils and essential oil components towards oral bacteria. Oral Microbiol Immunol 1994;9:202-8.
(24) Tirillini B, Velasquez ER, Pellegrino R. Chemical composition and antimicrobial activity of essential oil of Piper angustifolium [letter]. Planta Med 1996;62:372-3.
(25) Vargas I, Sanz I, Moya P, Prima-Yufera E. Antimicrobial and antioxidant compounds in the nonvolatile fraction of expresed orange essential oil. J Food Prot 1999;62:929-32.
(26) Vila R, Valenzuela L, Bello H, Canigueral S, Montes M, Adzet T. Composition and antimicrobial activity of the essential oil of Peumus boldus leaves [letter]. Planta Med 1999;65:178-9.
(27) Inouye S, Takizawa T., Yamaguchi H. Antibacterial activity of essential oils and their major constituents against respiratory tract pathogens by gaseous contact. Journal of Antimicrobial Chemotherapy (2001) 47, 565-573.

## Claims

1. A delivery system adapted to be inserted into the external ear canal comprising a casing of a vapour impermeable material containing a volatile substance with therapeutic effect on otitis media, wherein the casing has a vapour-permeable opening, said the vapour-permeable opening being sealed off with a liquid-impermeable and vapour-permeable film.

2. A delivery system according to claim 1 further comprising a carrier comprising the volatile substance.

3. A delivery system according to claim 2, wherein the carrier is made of cotton.

4. A delivery system according to any of claims 1-3, wherein the vapour-permeable opening is sealed off with a removable vapour-impermeable film.

5. A delivery system according to any of the preceding claims, wherein the casing has one or more protrusions adapted to help keep the delivery system in place after insertion into the external ear canal.

6. A delivery system according to claim 5, wherein the protrusion is an annular protrusion.

7. A delivery system according to any of the preceding claims, wherein the casing has a tapered head at the end comprising the vapour-permeable opening.

8. A delivery system according to any of the preceding claims, wherein the casing is made from a rubber material.

9. A delivery system according to any of the preceding claims, wherein the casing is made from a silicone material.

10. A delivery system according to any of the preceding claims, wherein the volatile substance is an essential oil.

11. A delivery system according to claim 10, wherein the essential oil is oil of Basil (Ocimum Basilicum).

12. A delivery system according to any of the preceding claims, wherein the casing is oblong.

13. A delivery system according to claim 12, wherein the vapour-permeable opening is provided at one end of the casing.

14. A delivery system according to any of the preceding claims, wherein the casing has a circular cross-section.

## Patentansprüche

1. Bereitstellungssystem, geeignet zum Einführen in den äußeren Gehörgang, umfassend ein Gehäuse aus einem dampfundurchlässigen Material, welches eine flüchtige Substanz mit therapeutischer Wirkung bei Mittelohrentzündung enthält, wobei das Gehäuse eine dampfdurchlässige Öffnung aufweist, wobei die dampfdurchlässige Öffnung mit einer flüssigkeitsundurchlässigen und dampfdurchlässigen Folie abgedichtet ist.

2. Bereitstellungssystem nach Anspruch 1, ferner umfassend einen Träger, welcher die flüchtige Substanz enthält.

3. Bereitstellungssystem nach Anspruch 2, wobei der Träger aus Baumwolle hergestellt ist.

4. Bereitstellungssystem nach einem der Ansprüche 1-3, wobei die dampfdurchlässige Öffnung mit einer entfernbaren dampfundurchlässigen Folie abgedichtet ist.

5. Bereitstellungssystem nach einem der vorhergehenden Ansprüche, wobei das Gehäuse einen oder mehrere Vorsprünge aufweist, die dazu geeignet sind, das Bereitstellungssystem nach dem Einführen in den äußeren Gehörgang an Ort und Stelle zu halten.

6. Bereitstellungssystem nach Anspruch 5, wobei der Vorsprung ein ringförmiger Vorsprung ist.

7. Bereitstellungssystem nach einem der vorhergehenden Ansprüche, wobei das Gehäuse einen kegelförmigen Kopf an dem Ende aufweist, welches die dampfdurchlässige Öffnung umfasst.

8. Bereitstellungssystem nach einem der vorhergehenden Ansprüche, wobei das Gehäuse aus einem Gummimaterial hergestellt ist.

9. Bereitstellungssystem nach einem der vorhergehenden Ansprüche, wobei das Gehäuse aus einem Silikonmaterial hergestellt ist.

10. Bereitstellungssystem nach einem der vorhergehenden Ansprüche, wobei die flüchtige Substanz ein ätherisches Öl ist.

11. Bereitstellungssystem nach Anspruch 10, wobei das ätherische Öl Basilikumöl (Ocimum Basilicum) ist.

12. Bereitstellungssystem nach einem der vorhergehenden Ansprüche, wobei das Gehäuse länglich ist.

13. Bereitstellungssystem nach Anspruch 12, wobei die dampfdurchlässige Öffnung an einem Ende des Gehäuses bereitgestellt ist.

14. Bereitstellungssystem nach einem der vorhergehenden Ansprüche, wobei das Gehäuse einen kreisförmigen Querschnitt aufweist.

## Revendications

1. Système de délivrance apte à être inséré dans le canal auriculaire externe, comprenant un boîtier en matériau imperméable à la vapeur contenant une substance volatile ayant un effet thérapeutique sur les agents de l'otite, ce boîtier ayant une ouverture perméable à la vapeur, ladite ouverture perméable à la vapeur étant scellée avec un film imperméable au liquide et perméable à la vapeur.

2. Système de délivrance selon la revendication 1, comprenant en plus un support comportant la substance volatile.

3. Système de délivrance selon la revendication 2, le support étant en coton.

4. Système de délivrance selon l'une quelconque des revendications 1 à 3, l'ouverture perméable à la vapeur étant scellée avec un film amovible imperméable à la vapeur.

5. Système de délivrance selon l'une quelconque des revendications précédentes, le casier comportant une ou plusieurs saillies aptes à maintenir le système de délivrance en place après insertion dans le canal auriculaire externe.

6. Système de délivrance selon la revendication 5, la saillie étant une saillie annulaire.

7. Système de délivrance selon l'une quelconque des revendications précédentes, le casier ayant une tête effilée à l'extrémité comportant l'ouverture perméable à la vapeur.

8. Système de délivrance selon l'une quelconque des revendications précédentes, le casier étant en matériau de caoutchouc.

9. Système de délivrance selon l'une quelconque des revendications précédentes, le casier étant en matériau de silicone.

10. Système de délivrance selon l'une quelconque des revendications précédentes, la substance volatile étant une huile essentielle.

11. Système de délivrance selon l'une quelconque des revendications précédentes, l'huile essentielle étant de l'huile de basilic (ocimum basilicum).

12. Système de délivrance selon l'une quelconque des revendications précédentes, le casier étant oblong.

13. Système de délivrance selon la revendication 12, l'ouverture perméable à la vapeur étant prévue à une extrémité du casier.

14. Système de délivrance selon l'une quelconque des revendications précédentes, le casier ayant une section transversale circulaire.
